# FASCICULE DE BREVET EUROPEEN

(11) **EP 1 757 328 B1**
(45) Date de publication et mention de la délivrance du brevet: **12.08.2009**
(21) Numéro de dépôt: 06291350.4
(22) Date de dépôt: 24.08.2006
(51) Int. Cl.: A61Q 5/10, A61K 8/04, A61K 8/37, A61K 8/22, A61K 8/23, A61K 8/81, A61K 8/87, A61K 8/88, A61K 8/85, A61K 8/41, A61K 8/33, A61K 8/34, A61K 8/42, A61K 8/66, A61K 8/89

(54) **Composition tinctoriale comprenant des composés insolubles, procédés mettant en oeuvre cette composition.**
Haarfärbemittel enthaltend unlösliche Derivate und Verfahren
Hair dye compositions comprising insolubles compounds and processses

(30) Priorité: 25.08.2005 FR 0508753
(43) Date de publication de la demande: 28.02.2007
(73) Titulaire: L'Oréal, 75008 Paris (FR)
(72) Inventeur: Kravtchenko, Sylvain, Shanghai 200021 (CN); Dubief, Claude, 78150 Le Chesnay (FR)
(74) Mandataire: Casalonga, Axel

(56) Documents cités:
- EP-A- 0 823 250
- WO-A-20/05074871
- US-A1- 2005 081 311
- US-B1- 6 254 647
- US-B1- 6 277 154
- DATABASE WPI Section Ch, Week 200415 Derwent Publications Ltd., London, GB; Class D21, AN 2004-149220 XP002380459 & JP 2004 026703 A (HOYU KK) 29 janvier 2004 (2004-01-29)

## Description

La présente invention a trait à des compositions tinctoriales des matières kératiniques comprenant un composé insoluble dans l'eau, à un procédé de teinture des fibres kératiniques humaines et en particulier des cheveux et à l'utilisation de ladite composition pour la coloration capillaire.

Il est connu de teindre les fibres kératiniques humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser poser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliqué à pH basique, ce procédé permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est-à-dire de la rendre plus visible.

Il est aussi connu de teindre les fibres kératiniques humaines par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Il est connu par exemple d'utiliser des colorants directs nitrés benzèniques, anthraquinoniques, nitropyridiniques, des colorants azoïques, xanthéniques, acridiniques aziniques ou des colorants triarylméthaniques.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Il est connu d'utiliser des colorants directs en combinaison avec des agents oxydants. Cependant, les colorants directs sont généralement sensibles à l'action des agents oxydants tels que l'eau oxygénée, et les agents réducteurs tels que le bisulfite de sodium, ce qui les rend généralement difficilement utilisables dans les compositions de teinture directe éclaircissante à base d'eau oxygénée et à base d'un agent alcalinisant ou dans des compositions de teinture d'oxydation en association avec des précurseurs du type bases d'oxydation ou coupleurs. Il a été proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pose des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

L'utilisation de corps gras est par ailleurs connue dans le domaine de la coloration capillaire.

La demande EP 0 823 250 décrit des émulsions à inversion de phase en deux parties pour le traitement des cheveux, la première partie pouvant comprendre un colorant d'oxydation et une huile polaire telle que l'alcool isostéarylique et la seconde partie comprenant un agent oxydant.

La demande JP 2004-026703 décrit également des émulsions à inversion de phase pouvant être utilisées pour la teinture ou la décoloration des cheveux, obtenues par mélange d'une première composition comprenant une huile, un tensio-actif, de l'eau et un colorant et d'une seconde composition comprenant un agent oxydant.

La demande US 2005/0081311 et la demande WO 2005/074871 décrivent des compositions de teinture des cheveux leur conférant un effet nacré grâce à la combinaison d'alcools gras, d'alcanolamides, d'alcools gras éthoxylés avec des précurseurs de colorants d'oxydation et/ou des colorants directs. Ces compositions sont mélangées juste avant emploi avec un agent oxydant.

Les demandes de brevet US 6,277,154 et 6,254,647 décrivent des procédés de préparation de compositions de teinture des cheveux faciles à mélanger avec les agents oxydants, due gagner du temps et de l'énergie, consistant respectivement à préparer dans un premier temps une pré-émulsion comprenant des gras tels que des alcools gras ou à préparer une émulsion stable comprenant des corps gras solides et des corps gras liquides, et à mélanger ensuite ces émulsions avec une composition aqueuse comprenant au moins un précurseur de colorant d'oxydation. L'agent oxydant est ajouté à l'émulsion colorante au moment de l'application sur les cheveux.

Le temps de pose d'une composition de coloration d'oxydation ou directe éclaircissante est classiquement compris entre 15 et 45 minutes selon la nature de la fibre (sensibilisée ou non) et la nature du colorant utilisée. Depuis longtemps, des recherches sont menées pour diminuer le temps de pose des compositions colorantes, sans pour autant augmenter la concentration des constituants et tout en conservant un bon niveau de coloration, c'est-à-dire une bonne puissance tinctoriale, une bonne tenue de la couleur face aux agents extérieurs et dans le temps.

Il existe un réel besoin de disposer de compositions de coloration d'oxydation ou directe éclaircissante améliorées en termes d'efficacité, de rapidité de réaction ou de pénétration du colorant dans la fibre, tout en conservant une bonne innocuité, une bonne ténacité et une bonne sélectivité, cette dernière résultant de la différence de montée entre différentes parties d'un cheveu ou d'une chevelure.

De manière surprenante et avantageuse, la Demanderesse vient de découvrir que l'utilisation d'une composition comprenant au moins un précurseur de colorant d'oxydation ou un colorant direct, au moins un agent oxydant et au moins un composé organique oxygéné non colorant insoluble dans l'eau pour la teinture des fibres kératiniques tel que défini à la revendication 1, en particulier des fibres kératiniques humaines, telles que les cheveux, permet ces améliorations.

Outre leur avantage en terme d'innocuité, ces compositions permettent une application de ladite composition sur les fibres à colorer plus courte, et ainsi un contact plus court avec les agents oxydants, qui ont tendance à détériorer la fibre et permettent l'obtention de reflets homogènes et tenaces.

En outre, ces compositions présentent un bon profil toxicologique.

L'invention concerne également les procédés de teinture d'oxydation et d'éclaircissement des fibres kératiniques et l'utilisation de ladite composition pour la coloration desdites fibres.

D'autres caractéristiques, aspects, objets et avantages de l'invention figurant dans la description ci-dessous permettront de mieux cerner l'invention.

La présente invention a donc pour objet une composition tinctoriale aqueuse pour la teinture des fibres kératiniques, en particulier des fibres kératiniques humaines telles que les cheveux comprenant, dans un milieu de teinture approprié :
(a) au moins un agent oxydant,
(b) au moins 30% en poids d'au moins un composé organique oxygéné non colorant insoluble dans l'eau par rapport au poids total de ladite composition tel que défini à la revendication 1, et
(c) au moins un précurseur de colorant d'oxydation et/ou un colorant direct.

L'agent oxydant est choisi de préférence parmi le peroxyde d'hydrogène et les composés susceptibles de produire par hydrolyse du peroxyde d'hydrogène, tels que le peroxyde d'urée, les bromates de métaux alcalins, les persels tels que les perborates, persulfates, percarbonates, et peroxydes de métaux alcalins ou alcalino-terreux, comme le sodium, le potassium, le magnésium, les peracides ou leurs mélanges ; et les enzymes oxydases parmi lesquelles on peut citer les peroxydases, les oxydo-réductases à 2 électrons telles que les uricases et les oxygénases à 4 électrons comme les laccases, le cas échéant en présence de leur substrat approprié. Le peroxyde d'hydrogène est particulièrement préféré.

La teneur en agent oxydant est comprise entre 2 et 35% en poids de la composition tinctoriale, de préférence entre 5 et 30 % en poids de la composition tinctoriale.

Par composé organique oxygéné, on entend au sens de la présente invention tout composé organique comportant au moins un atome d'oxygène dans sa structure moléculaire élémentaire.

Par composé organique oxygéné non colorant insoluble dans l'eau selon l'invention, on entend des composés oxygénés présentant une solubilité dans l'eau à température ambiante (25°C) inférieure à 0.5 % en poids. Les composés organiques oxygénés de l'invention peuvent être des composés polymériques ou non polymériques.

Les composés organiques oxygénés de l'invention sont des composés non polymériques.

choisis parmi les alcools gras et les esters d'acides ou d'alcools. Concernant les esters d'acides ou d'alcools gras, les chaînes grasses des acides ou alcools gras comportent de 8 à 40 atomes de carbone et sont éventuellement hydroxydés. Les alcools gras comportent de 8 à 40 atomes de carbone. A titre d'exemple, citons : le mono et le distéarate d'éthylène glycol, le pentaerythritol monooléate, le sorbitan tristéarate, le dioléate de glycérol, les esters gras d'éthylène glycol ou de propylène glycol, l'alcool stéarylique, l'alcool béhénylique, l'alcool cétylstéarylique.

La teneur en composé organique oxygéné insoluble dans l'eau est supérieure à 30% en poids de la composition tinctoriale, de préférence est comprise entre 30 et 75 % en poids de la composition tinctoriale, et plus particulièrement est comprise entre 30 et 60 % en poids de la composition tinctoriale.

Ainsi lorsque que le ou les composés insolubles dans l'eau selon l'invention sont solides, la composition est sous forme d'une suspension. Lorsque le ou les composés insolubles dans l'eau selon l'invention sont sous forme liquide, par exemple sous forme d'une phase organique insoluble dans la phase aqueuse, la composition est une émulsion, voire une dispersion.

La composition de la présente invention peut comprendre également au moins un précurseur de colorant d'oxydation.

A titre d'exemple, les précurseurs de colorant d'oxydation sont choisis parmi les phénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidiné, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La ou les précurseurs de colorant d'oxydation présents dans la composition de l'invention sont en général présentes en quantité comprise allant de 0,001 à 20 % en poids environ du poids total de la composition tinctoriale, de préférence allant de 0,005 à 6 %.

La composition tinctoriale conforme à l'invention peut en outre contenir un ou plusieurs colorants directs choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1 -amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO 95/01772, EP-714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100 369 et FR 2 844 269, dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7.

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1 ,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20 % en poids environ du poids total de la composition, et encore plus préférentiellement de 0,005 à 10 % en poids environ.

Si la composition contient au moins un précurseur de colorant d'oxydation, la composition selon l'invention contient de préférence un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, les monoéthers de polyol tels que le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La composition selon l'invention contient de 0,1 à 69 % d'eau, de préférence de 10 à 68 %, plus préférentiellement de 20 à 65 %, et encore plus préférentiellement de 30 à 60 % en poids environ du poids total de la composition tinctoriale.

Les solvants organiques sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges autres que ceux de l'invention, des agents épaississants minéraux ou organiques, et en particulier les épaississants associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs et des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12, de préférence entre 5 et 11, et encore plus particulièrement de 6 à 8,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines, telles que les mono-, di- et triéthanolamines ainsi que leurs dérivés, les hydroxydes de sodium ou de potassium et les composés de formule (II) suivante : 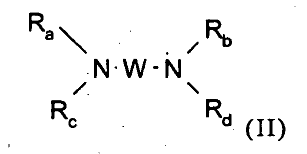 dans laquelle W est un reste propylène éventuellement substitué par un groupement hydroxyle ou un radical alkyle en C₁-C₄; Rₐ, R_{b}, R_{c} et R_{d}, identiques ou différents, représentent un atome d'hydrogène, un radical alkyle en C₁-C₄ ou hydroxyalkyle en C₁-C₄.

La composition selon l'invention peut se trouver sous sa forme ou peut résulter du mélange extemporanée de 2 compositions ou plus. Par exemple, la composition peut être obtenue à partir du mélange d'une composition comprenant l'agent oxydant et d'une composition comprenant le ou les composés organiques oxygénés insolubles dans l'eau et le ou les colorants.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains. Il en est de même pour les compositions, qui suite à leur mélange conduisent à la composition selon l'invention, sous réserve que l'une d'entre elles soit une solution aqueuse.

Le procédé de la présente invention est un procédé dans lequel on applique sur les fibres la composition selon la présente invention telle que définie précédemment, puis on rince lesdites fibres.

De préférence, le procédé comprend les étapes d'appliquer la composition tinctoriale sur les fibres kératiniques, puis à laisser poser pendant une période comprise entre 3 minutes et 1 heure et de préférence entre 15 minutes et 45 minutes, puis on rince lesdites fibres.

Ainsi, lorsque la composition selon l'invention utilisée dans le procédé de coloration comprend, en tant que colorant, qu'un ou des précurseurs de colorant d'oxydation, éventuellement combiné à des coupleurs, le procédé selon l'invention est un procédé de coloration d'oxydation.

Lorsque la composition selon l'invention utilisée dans le procédé de coloration ne comprend pas de précurseurs de colorant d'oxydation, mais uniquement un ou des colorants directs, le procédé selon l'invention est un procédé de coloration directe éclaircissante.

L'invention porte également sur l'utilisation de la composition tinctoriale selon l'invention pour la teinture des fibres kératiniques.

## Revendications

1. Composition tinctoriale aqueuse pour la teinture des fibres kératiniques, en particulier des fibres kératiniques humaines telles que les cheveux comprenant, dans un milieu de teinture approprié :
(a) au moins un agent oxydant,
(b) au moins 30% en poids par rapport au poids total de ladite composition d'au moins un composé organique oxygéné non-colorant insoluble dans l'eau, et c'est-à-dire, présentant une solubilité dans l'eau à température ambiante (25°C) inférieure à 0.5 % en poids les composés organiques oxygénés insolubles dans l'eau étant de nature non polymérique sont choisis parmi les alcools gras dont la chaîne grasse est en C₈-C₄₀, les esters, d'acides gras ou d'alcool gras ; les chaînes grasses des acides ou alcools gras comportant de 8 à 40 atomes de carbone; et
(c) au moins un précurseur de colorant d'oxydation et/ou un colorant direct.

2. Composition selon la revendication 1, **caractérisée en ce que** l'agent oxydant est choisi parmi le peroxyde d'hydrogène, le peroxyde d'urée, les bromates de métaux alcalins, les perborates, persulfates, percarbonates, et peroxydes de métaux alcalins ou alcaline-terreux, les peracides ou leurs mélanges ; et les enzymes oxydases.

3. Composition selon la revendication 2, **caractérisée en ce que** l'agent oxydant est le peroxyde d'hydrogène.

4. Composition selon l'une quelconque des revendications i à 3,
**caractérisée en ce que** la teneur en agent oxydant est comprise entre 2 et 35% en poids de la composition tinctoriale, de préférence entre 5 et 30 % en poids de la composition tinctoriale.

5. Composition selon l'une quelconque des revendications 1 à 4, **caractérisée en ce que** les composées organiques insolubles dans l'eau sont choisis parmi le mono et le di-stéatate d'éthylène glycol, le monooléate de pentaerythritol, le tristéarate de sorbitan, le dioléate de glycérol, les esters gras d'éthylène glycol ou de propylène glycol, l'alcool stéarylique, l'alcool béhénylique, l'alcool cétylstéarylique.

6. Composition selon l'une quelconque des revendication 1 à 5, **caractérisée en ce que** la teneur en composé organique oxygéné insoluble dans l'eau est supérieure à 30% en poids de la composition tinctoriale, de préférence est comprise entre 30 et 90 % en poids de la composition tinctoriale, et plus particulièrement est comprise entre 30 et 60% en poids de la composition tinctoriale.

7. Composition selon l'une quelconque des revendications 1 à 6, **caractérisée en ce que** le précurseur de colorant d'oxydation est choisi parmi les para-phénylènediamines, les bisphénylalkylènediamines, les para-aminophénols, les ortho-aminophénols, les bases hétérocycliques et leurs sels d'addition.

8. Composition selon la revendication 7, **caractérisée en ce que** le ou les précurseurs d'oxydation sont présentes en une quantité comprise entre 0,001 à 20 % en poids et de préférence entre 0,005 et 6% en poids par rapport an poids total de la composition.

9. Composition selon l'une des revendications 1 à 8,
**caractérisée en ce que** le colorant direct est choisi parmi les colorants nitres de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

10. Composition selon la revendication 9, **caractérisée en ce que** le ou les colorants directs, sont présents en une quantité comprise entre 0,001 à 20 % en poids et de préférence entre 0,005 et 10% en poids par rapport au poids total de la composition.

11. composition selon l'une des revendications 1 à 10.
**caractérisée en ce qu'**elle comprend en outre un coupleur choisi parmi les méta-phénylènediamines, les méta-aminophénols, les métadiphénols les coupleurs naphtaléniques, les coupleurs hétérocycliques et leurs sels d'addition.

12. Composition selon la revendication 11 **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4. méthylène dioxybenzène, le 2,6-bis-(β-bydroxyéthylamino)toluène et leurs sels d'addition.

13. Composition selon la revendication 11 ou 12, **caractérisée en ce que** le ou les coupleurs sont présents et une quantité comprise entre 0,001 et 20 %, de préférence entre 0,005 et 6 % en poids par rapport au poids total de la composition.

14. Composition selon l'une, quelconque des revendications 1 à 13, telle qu'elle comprend au moins un solvant organique tel que l'éthanol, le propylène glycol, le glycérol, les mono éthers de Polyols.

15. Composition selon l'une quelconque des revendications 1 à 14, telle qu'elle contient 0,1 à 69 % d'eau, de préférence 10 à 68 %, plus préférentiellement 20 à 65 % et en particulier 30 à 60 % en poids par rapport au poids total de la composition.

16. Composition selon l'une quelconque des revendications 1 à 15, **caractérisée en ce qu'**elle comprend au moins un adjuvant choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges autres que ceux définis ci-dessus, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents conditionneurs tels que les silicones volatiles ou non volatiles, modifiées ou non modifiées, les agents filmogènes, les céramides, les agents conservateurs, les agents opacifiants.

17. Procédé de teinture des fibres kératiniques, tel qu'il comprend les étapes d'appliquer la composition tinctoriale selon l'une des revendications 1 à 16 sur les fibres kératiniques, puis à laisser poser pendant une période comprise entre 3 minutes et 1 heure et de préfèrence entre 15 minutes et 45 minutes, puis on rince lesdites fibres.

18. Utilisation d'une composition selon l'une des revendications 1 à 16 pour la teinture des fibres kératiniques.

## Claims

1. Aqueous dye composition for dyeing keratin fibres, in particular human keratin fibres such as the hair, comprising, in a suitable dyeing medium:
(a) at least one oxidizing agent,
(b) at least 30% by weight, relative to the total weight of the said composition, of at least one water-insoluble, non-colouring, oxygenated organic compound, that is to say with a solubility in water at room temperature (25°C) of less than 0.5% by weight, the water-insoluble oxygenated organic compounds being of non-polymeric nature and being chosen from fatty alcohols with a C₈-C₄₀ fatty chain, and fatty acid or fatty alcohol esters; the fatty chains of the fatty acids or fatty alcohols containing from 8 to 40 carbon atoms; and
(c) at least one oxidation dye precursor and/or one direct dye.

2. Composition according to Claim 1, **characterized in that** the oxidizing agent is chosen from hydrogen peroxide, urea peroxide, alkali metal bromates, alkali metal or alkaline-earth metal perborates, persulfates, percarbonates or peroxides, and peracids, or mixtures thereof; and oxidase enzymes.

3. Composition according to Claim 2, **characterized in that** the oxidizing agent is hydrogen peroxide.

4. Composition according to any one of Claims 1 to 3, **characterized in that** the content of oxidizing agent is between 2% and 35% by weight of the dye composition and preferably between 5% and 30% by weight of the dye composition.

5. Composition according to any one of Claims 1 to 4, **characterized in that** the water-insoluble organic compounds are chosen from ethylene glycol monostearate and distearate, pentaerythrityl monooleate, sorbitan tristearate, glyceryl dioleate, fatty esters of ethylene glycol or of propylene glycol, stearyl alcohol, behenyl alcohol and cetylstearyl alcohol.

6. Composition according to any one of Claims 1 to 5, **characterized in that** the content of water-insoluble oxygenated organic compound is greater than 30% by weight of the dye composition and preferably between 30% and 90% by weight of the dye composition, and is more particularly between 30% and 60% by weight of the dye composition.

7. Composition according to any one of Claims 1 to 6, **characterized in that** the oxidation dye precursor is chosen from para-phenylenediamines, bis(phenyl)alkylenediamines, para-aminophenols, ortho-aminophenols and heterocyclic bases, and the addition salts thereof.

8. Composition according to Claim 7, **characterized in that** the oxidation dye precursor(s) is (are) present in an amount of between 0.001% and 20% by weight and preferably between 0.005% and 6% by weight relative to the total weight of the composition.

9. Composition according to one of Claims 1 to 8, **characterized in that** the direct dye is chosen from neutral, acidic or cationic nitrobenzene dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

10. Composition according to Claim 9, **characterized in that** the direct dye(s) is (are) present in an amount of between 0.001% and 20% by weight and preferably between 0.005% and 10% by weight relative to the total weight of the composition.

11. Composition according to one of Claims 1 to 10, **characterized in that** it also comprises a coupler chosen from meta-phenylenediamines, meta-aminophenols, meta-diphenols, naphthalene-based couplers and heterocyclic couplers, and the addition salts thereof.

12. Composition according to Claim 11, **characterized in that** the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

13. Composition according to Claim 11 or 12, **characterized in that** the coupler(s) is (are) present in an amount of between 0.001% and 20% and preferably between 0.005% and 6% by weight relative to the total weight of the composition.

14. Composition according to any one of Claims 1 to 13, such that it comprises at least one hydroxylated solvent such as ethanol, propylene glycol, glycerol or polyol monoethers.

15. Composition according to any one of Claims 1 to 14, such that it contains 0.1% to 69%, preferably 10% to 68%, more preferentially 20% to 65% and in particular 30% to 60% by weight of water relative to the total weight of the composition.

16. Composition according to any one of Claims 1 to 15, **characterized in that** it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof other than those defined above, mineral or organic thickeners, and in particular anionic, cationic, nonionic and amphoteric polymeric associative thickeners, antioxidants, penetrants, sequestrants, fragrances, buffers, dispersants, conditioning agents such as volatile or non-volatile, modified or unmodified silicones, film-forming agents, ceramides, preserving agents and opacifiers.

17. Process for dyeing keratin fibres, such that it comprises the steps of applying the dye composition according to one of Claims 1 to 16 to the keratin fibres, and then in leaving it to act for a period of between 3 minutes and 1 hour and preferably between 15 minutes and 45 minutes, and the said fibres are then rinsed.

18. Use of a composition according to one of Claims 1 to 16 for dyeing keratin fibres.

## Patentansprüche

1. Wässrige Farbmittelzusammensetzung zum Färben von Keratinfasern und insbesondere menschlichen Keratinfasern, wie Haaren, die in einem zum Färben geeigneten Medium enthält:
(a) mindestens ein Oxidationsmittel,
(b) bezogen auf das Gesamtgewicht der Zusammensetzung mindestens 30 Gew.-% mindestens einer in Wasser unlöslichen, nicht färbenden, sauerstoffhaltigen organischen Verbindung, d.h. die in Wasser bei Raumtemperatur (25 °C) eine Löslichkeit unter 0,5 Gew.-% aufweist, wobei die in Wasser unlöslichen, sauerstoffhaltigen organischen Verbindungen von der Art der nichtpolymeren Verbindungen sind und unter den Fettalkoholen, deren Fettkette 8 bis 40 Kohlenstoffatome aufweist, und den Estern von Fettsäuren oder Fettalkoholen ausgewählt sind, wobei die Fettketten der Fettsäuren oder Fettalkohole 8 bis 40 Kohlenstoffatome aufweisen, und
(c) mindestens ein Farbstoffvorprodukt eines Oxidationsfarbstoffes und/oder einen Direktfarbstoff.

2. Zusammensetzung nach Anspruch 1, **dadurch gekennzeichnet, dass** das Oxidationsmittel unter Wasserstoffperoxid, Harnstoffperoxid, Alkalimetallbromaten, Perboraten, Persulfaten, Percarbonaten und Peroxiden von Alkalimetallen oder Erdalkalimetallen, Persäuren oder deren Gemischen; und Oxidasen ausgewählt ist.

3. Zusammensetzung nach Anspruch 2, **dadurch gekennzeichnet, dass** es sich bei dem Oxidationsmittel um Wasserstoffperoxid handelt.

4. Zusammensetzung nach einem der Ansprüche 1 bis 3, **dadurch gekennzeichnet, dass** der Mengenanteil des Oxidationsmittels im Bereich von 2 bis 35 Gew.-% der Farbmittelzusammensetzung und vorzugsweise im Bereich von 5 bis 30 Gew.-% der Farbmittelzusammensetzung liegt.

5. Zusammensetzung nach einem der Ansprüche 1 bis 4, **dadurch gekennzeichnet, dass** die in Wasser unlöslichen, organischen Verbindungen unter Ethylenglycolmono- und -distearat, Pentaerythritmonooleat, Sorbitantristearat, Glycerindioleat, Fettsäureestern von Ethylenglycol oder Propylenglycol, Stearylalkohol, Behenylalkohol und Cetylstearylalkohol ausgewählt sind.

6. Zusammensetzung nach einem der Ansprüche 1 bis 5, **dadurch gekennzeichnet, dass** der Mengenanteil der in Wasser unlöslichen, sauerstoffhaltigen, organischen Verbindung mehr als 30 Gew.-% der Farbmittelzusammensetzung beträgt und vorzugsweise im Bereich von 30 bis 90 Gew.-% der Farbmittelzusammensetzung und insbesondere im Bereich von 30 bis 60 Gew.-% der Farbmittelzusammensetzung liegt.

7. Zusammensetzung nach einem der Ansprüche 1 bis 6, **dadurch gekennzeichnet, dass** das Farbstoffvorprodukt eines Oxidationsfarbstoffes unter den *para*-Phenylendiaminen, Bisphenylalkylendiaminen, *para*-Aminophenolen, *ortho*-Aminophenolen, heterocyclischen Basen und deren Additionssalzen ausgewählt ist.

8. Zusammensetzung nach Anspruch 7, **dadurch gekennzeichnet, dass** das oder die Farbstoffvorprodukte von Oxidationsfarbstoffen in einer Menge von 0,001 bis 20 Gew.-% und vorzugsweise 0,005 bis 6 Gew.-%, bezogen auf das Gesamtgewicht der Zusammensetzung, enthalten sind.

9. Zusammensetzung nach einem der Ansprüche 1 bis 8, **dadurch gekennzeichnet, dass** der Direktfarbstoff unter den neutralen, sauren oder kationischen, nitrierten Direktfarbstoffen auf Benzolbasis, neutralen, sauren oder kationischen, direktziehenden Azofarbstoffen, neutralen, sauren oder kationischen direktziehenden Chinonfarbstoffen und insbesondere AnthrachinonFarbstoffen, direktziehenden Azinfarbstoffen, direktziehenden Triarylmethanfarbstoffen, direktziehenden Indoaminfarbstoffen und direktziehenden natürlichen Farbstoffen ausgewählt ist.

10. Zusammensetzung nach Anspruch 9, **dadurch gekennzeichnet, dass** der oder die Direktfarbstoffe in einer Menge von 0,001 bis 20 % und vorzugsweise 0,005 bis 10 %, bezogen auf das Gesamtgewicht der Zusammensetzung, enthalten sind.

11. Zusammensetzung nach einem der Ansprüche 1 bis 10, **dadurch gekennzeichnet, dass** sie ferner einen Kuppler enthält, der unter den *meta*-Phenylendiaminen, *meta*-Aminophenolen, *meta-*Dihydroxybenzolen, Naphthalinkupplern, heterocyclischen Kupplern und deren Additionssalzen ausgewählt ist.

12. Zusammensetzung nach Anspruch 11, **dadurch gekennzeichnet, dass** der Kuppler unter 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxypyridin, 6-Hydroxybenzomorpholin, 3,5-Diamino-2,6-dimethoxypyridin, 1-N-(β-Hydroxyethyl)amino-3,4-methylendioxybenzol, 2,6-Bis(β-hydroxyethylamino)toluol und ihren Additionssalzen ausgewählt ist.

13. Zusammensetzung nach Anspruch 11 oder 12, **dadurch gekennzeichnet, dass** der oder die Kuppler in einer Menge von 0,001 bis 20 Gew.-% und vorzugsweise 0,005 bis 6 Gew.-%, bezogen auf das Gesamtgewicht der Zusammensetzung, enthalten sind.

14. Zusammensetzung nach einem der Ansprüche 1 bis 13, wobei sie mindestens ein organisches Lösemittel enthält, wie Ethanol, Propylenglycol, Glycerol, Monoether von Polyolen.

15. Zusammensetzung nach einem der Ansprüche 1 bis 14, wobei sie 0,1 bis 69 Gew.-% Wasser, vorzugsweise 10 bis 68 Gew.-% Wasser, noch bevorzugter 20 bis 65 Gew.-% Wasser und insbesondere 30 bis 60 Gew.-% Wasser, bezogen auf das Gesamtgewicht der Zusammensetzung, enthält.

16. Zusammensetzung nach einem der Ansprüche 1 bis 15, **dadurch gekennzeichnet, dass** sie mindestens einen Zusatzstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, die von den oben definierten Polymeren verschieden sind, anorganischen oder organischen Verdickungsmitteln und insbesondere anionischen, kationischen, nichtionischen, und amphoteren, assoziativen, polymeren Verdickungsmitteln, Antioxidantien, Penetrationsmitteln, Maskierungsmitteln, Parfums, Puffern, Dispergiermitteln, Konditioniermitteln, wie flüchtigen oder nicht flüchtigen, modifizierten oder nichtmodifizierten Siliconen, Filmbildnern, Ceramiden, Konservierungsmitteln und Trübungsmitteln ausgewählt ist.

17. Verfahren zum Färben von Keratinfasern, wobei es die Schritte umfasst, eine Farbmittelzusammensetzung nach einem der Ansprüche 1 bis 16 auf die Keratinfasern aufzutragen, anschließend während einer Zeitspanne von 3 Minuten bis 1 Stunde und vorzugsweise 15 bis 45 Minuten einwirken zu lassen und die Fasern dann zu spülen.

18. Verwendung einer Zusammensetzung nach einem der Ansprüche 1 bis 16 zum Färben von Keratinfasern.
